## Europäisches Patentamt

## European Patent Office

## Office européen des brevets

(11) Veröffentlichungsnummer: **0 144 528**

**A2**

(12) **EUROPÄISCHE PATENTANMELDUNG**

(21) Anmeldenummer: **84109624.1**

(22) Anmeldetag: **13.08.84**

(51) Int. Cl.⁴: **A 61 B 5/10**
**G 01 B 5/20**

(30) Priorität: **12.08.83 DE 8323274 U**

(43) Veröffentlichungstag der Anmeldung:
**19.06.85 Patentblatt 85/25**

(84) Benannte Vertragsstaaten:
**AT CH DE FR GB LI NL SE**

(71) Anmelder: **Cross, Lieselotte**
**Pulverstrasse 4**
**D-3062 Bückeburg(DE)**

(72) Erfinder: **Cross, Lieselotte**
**Pulverstrasse 4**
**D-3062 Bückeburg(DE)**

(74) Vertreter: **Eikenberg & Brümmerstedt Patentanwälte**
**Schackstrasse 1**
**D-3000 Hannover 1(DE)**

(54) **Vorrichtung zur Messung einer Fehlhaltung des Beckens, der Schulter und der Wirbelsäule des menschlichen Körpers.**

(57) Bei der Erfindung handelt es sich um eine Vorrichtung zur Messung einer funktionellen und/oder anatomischen Fehlhaltung des Beckens, der Schulter sowie der Wirbelsäule des menschlichen Körpers, die zwei vertikale Führungen im Abstand von mehr als einer Körperbreite enthält.

Auf den Führungen läuft jeweils mindestens eine höhenverschiebbare arretierbare Halterung, deren jeweilige Höhenstellung quantitativ erfaßbar ist.

Die Halterungen dienen als Aufnahme für horizontal in den Zwischenraum zwischen den Führungen hineinragende längsverschiebliche und arretierbare Taststäbe. Diese Taststäbe können in Längsrichtung federbelastet und in der Horizontalen schwenkbar sein, wobei diese Bewegungen ebenfalls quantitativ erfaßbar sein können, und sie dienen der Abtastung ausgewählter Bezugspunkte des Knochengerüstes.

Auf den Führungen können auch jeweils mehrere taststabbestückte Halterungen laufen.

Die Vorrichtung kann zur Ruhigstellung des Körpers und zur Erzielung definierter Grundstellungen mit Stütz- und Haltemitteln versehen sein.

./...

Fig. 1

0144528

— 1 —

Lieselotte Cross                                         516/1 EP


Vorrichtung zur Messung einer Fehlhaltung des
Beckens, der Schulter und der Wirbelsäule des
menschlichen Körpers

Fehlhaltungen des menschlichen Beckens können auf unterschiedliche Weise entstanden sein. Sie wirken sich auf das übrige
Knochengerüst, insbesondere die Wirbelsäule, die Schultern und
die Beine aus und bedürfen zumindest dann, wenn sie ein größeres
Ausmaß besitzen, einer Behandlung.

Die wohl häufigste Fehlhaltung ist eine Schiefstellung des
Beckens derart, daß in der Vorderansicht die eine Seite des Bekkens höher steht als die andere Seite. Diese Schiefstellung, die
meistens auch eine Verbiegung der Wirbelsäule und eine Schiefstellung der Schultern zur Folge hat, kann darauf beruhen, daß
ein Bein anatomisch verkürzt ist oder daß eine Anomalie (z. B.
eine Verwindung) des Beckens vorliegt, durch die ein Bein funktionell verkürzt erscheint. Beide Ursachen können auch gemeinsam
gegeben sein.

Bei einer anatomischen Beinverkürzung kann die Fehlhaltung
des Beckens nur auf mechanische Weise, insbesondere durch entsprechend ausgebildetes orthopädisches Schuhwerk ausgeglichen
werden, während sich die funktionelle Beinverkürzung durch spezielle krankengymnastische Maßnahmen behandeln läßt. Diese krankengymnastischen Maßnahmen können auch auf funktionelle Fehlhaltungen der Wirbelsäule und der Schulter erstreckt werden.

Das Problem bei solchen Fehlhaltungen bestand bisher darin, zum einen Art und Ausmaß der Fehlhaltung exakt zu messen und zum anderen bei der Behandlung, insbesondere einer krankengymnastischen Behandlung, den Erfolg der Therapie exakt zu kontrollieren. Durch bloßes Augenmaß ist dies nicht möglich, und auch eine Kontrolle durch Röntgenaufnahmen scheidet aus, weil hierbei rasch die zulässige Bestrahlungsdosis überschritten wird. Man ist deshalb auf mechanische Meßmethoden angewiesen.

In der Praxis am gebräuchlichsten ist derzeit ein Meßgerät, das in Form eines Gürtels oder eines U-förmigen Bügels um den Beckenbereich bzw. auf die Schultern des Patienten gelegt wird und eine Wasserwaage enthält, deren Libelle das Maß der Fehlhaltung anzeigt. Diese Meßmethode ist jedoch sehr grob und auch nur schlecht reproduzierbar, denn die Meßwerte hängen davon ab, wie genau das Gerät angelegt wird. Außerdem kann diese Meßmethode auch nicht zwischen einer anatomischen und einer funktionellen Beinverkürzung unterscheiden, sondern mißt die Summe beider Fehlhaltungen. Das ist unerwünscht, weil nur der funktionelle Fehlhaltungsanteil einer Therapie zugänglich ist.

Der Erfindung liegt die Aufgabe zugrunde, eine Vorrichtung zur Messung einer Fehlhaltung des Beckens, der Wirbelsäule und der Schulter des menschlichen Körpers zu schaffen, die genaue Meßwerte sowohl für den anatomischen als auch für den funktionellen Fehlhaltungsanteil liefert und eine reproduzierbare Kontrolle dieser Meßwerte während der Therapie ermöglicht, ohne daß der Körper einer Strahlenbelastung ausgesetzt wird.

Diese Aufgabe wird erfindungsgemäß dadurch gelöst, daß zwei vertikale Führungen im Abstand von mehr als einer Körperbreite vorgesehen sind, daß auf jeder Führung mindestens eine höhenverschiebbare Halterung angeordnet ist, deren jeweilige Höhenstellung quantitativ erfaßbar ist, und daß in jeder Halterung ein in den Zwischenraum zwischen den Führungen hineinragender horizontaler Taststab längsverschieblich gelagert ist.

- 3 -

Die erfindungsgemäße Vorrichtung ermöglicht eine völlig neuartige Meßmethode, die sowohl sehr schnell und einfach ausführbar ist als auch genaue und reproduzierbare Meßwerte liefert. Diese Meßmethode besteht darin, an den beiden gegenüberliegenden Seiten des Knochengerüstes eines aufrecht zwischen den Führungen stehenden Patienten mittels der Taststäbe korrespondierende Paare von ausgewählten Bezugspunkten abzutasten und die Höhenlage dieser Bezugspunkte zahlenmäßig zu erfassen. Aus der Differenz der Meßwerte für diese Bezugspunkte lassen sich dann eindeutige Rückschlüsse über Art und Ausmaß einer Fehlhaltung ableiten.

Bevorzugt werden dabei als Bezugspunkte jeweils links und rechts die beiden äußeren Rollhügel am Oberschenkelknochen (Trochanter major), die beiden seitlichen Oberkanten des Beckens und ggfs. noch die beiden Schulterblatt-Höhen ausgewählt, die nach-folgend kurz mit $T_l$, $T_r$, $B_l$, $B_r$, $S_l$ und $S_r$ bezeichnet sind. Die Messung dieser Bezugspunkte ist besonders aussagekräftig, denn die Differenz $(T_l - T_r)$ ist ein Maß für die anatomische Beinver-kürzung, während die Differenz $(B_l - B_r)$ die Gesamt-Fehlhaltung des Beckens angibt und somit aus der Differenz zwischen den Werten $(B_l - T_l)$ und $(B_r - T_r)$ die funktionelle Beinverkürzung ermittelt werden kann. Weiterhin läßt sich ausgehend davon, daß die Wirbelsäule senkrecht zum Becken und senkrecht zum Schultergürtel steht, beispielsweise aus der Differenz zwischen den Werten $(S_l - B_l)$ und $(S_r - B_r)$ auch Art und Ausmaß einer Verbiegung der Wirbelsäule erkennen.

Im allgemeinen genügt pro Meßvorgang eine Meßreihe zur Ermittlung dieser vorgenannten Differenzwerte, jedoch kann im Falle einer Überlagerung einer anatomischen mit einer funktionellen Beinverkürzung auch so vorgegangen werden, daß zunächst durch Ausgleichs-Unterlagen unter dem anatomisch verkürzten Bein die Differenz $(T_l - T_r)$ auf Null gebracht wird, bevor die übrigen Werte gemessen werden.

Im Prinzip läßt sich mit einem Taststab-Paar, mit dem aufeinanderfolgend die beiden Trochanter-Höhen, die beiden Becken-Höhen und ggfs. die beiden Schulterblatt-Höhen gemessen werden, bereits ein gutes Meßergebnis erzielen. Da jedoch unwillkürliche Bewegungen des Patienten während der Messung nicht auszuschließen sind, werden zweckmäßig pro Führung zwei oder drei Halterungen mit jeweils einem zugeordneten Taststab vorgesehen, die in der Meßstellung arretierbar sind. Dadurch kann beispielsweise mit einem ersten Taststab-Paar die Trochanter-Höhe gemessen und dieses Taststab-Paar dann in der Meßstellung belassen werden, um die Ruhighaltung des Patienten zu unterstützen, während mit dem nächsten Taststab-Paar das nächste Bezugspunkt-Paar angefahren wird. Bei Bedarf können auch noch zusätzliche Stütz- und Haltemittel zur Ruhighaltung des Körpers vorgesehen sein.

Die erfindungsgemäße Vorrichtung ist im übrigen nicht nur zur quantitativen Messung einer Schiefstellung des Beckens geeignet, sondern gestattet in einer vorteilhaften Weiterbildung auch, die Messung weiterer Fehlhaltungs-Typen, die bislang einer Messung kaum oder nicht zugänglich waren. Hierbei handelt es sich um die Beckenrotation (d.h. die Verdrehung des Beckens um die Vertikale) sowie um den horizontalen Beckenversatz. Dazu ist lediglich erforderlich, mindestens das zur Messung der Becken-Höhe vorgesehene Taststab-Paar um die Führung schwenkbar auszubilden und zusätzlich den Schwenkwinkel sowie den horizontalen Verschiebungsweg der beiden Taststäbe dieses Paares quantitativ zu erfassen. Nach Anfahren der Beckenoberkante von rechts und von links mit je einem Taststab ist dann aus der Winkeldifferenz Ausmaß und Richtung einer Rotation des Beckens zu ersehen und aus der Differenz der Taststab-Verschiebung Ausmaß und Richtung eines horizontalen Versatzes des Beckens. Bei dieser Weiterbildung der Erfindung sind die vorerwähnten zusätzlichen Stütz- und Haltemittel erforderlich, zweckmäßig in Form einer Kniestütze, an die der Patient herantreten muß, um reproduzierbare Meßbedingungen zu schaffen.

Die Erfindung wird nachfolgend in Ausführungsbeispielen anhand der Zeichnungen näher erläutert. Dabei stellen dar:

Fig. 1    eine einfache Ausführungsform der erfindungsgemäßen Vorrichtung,

Fig. 2    eine bevorzugte Ausführungsform der erfindungsgemäßen Vorrichtung, und

Fig. 3    einen Schnitt in der versetzten Ebene III-III
          der Fig. 2.

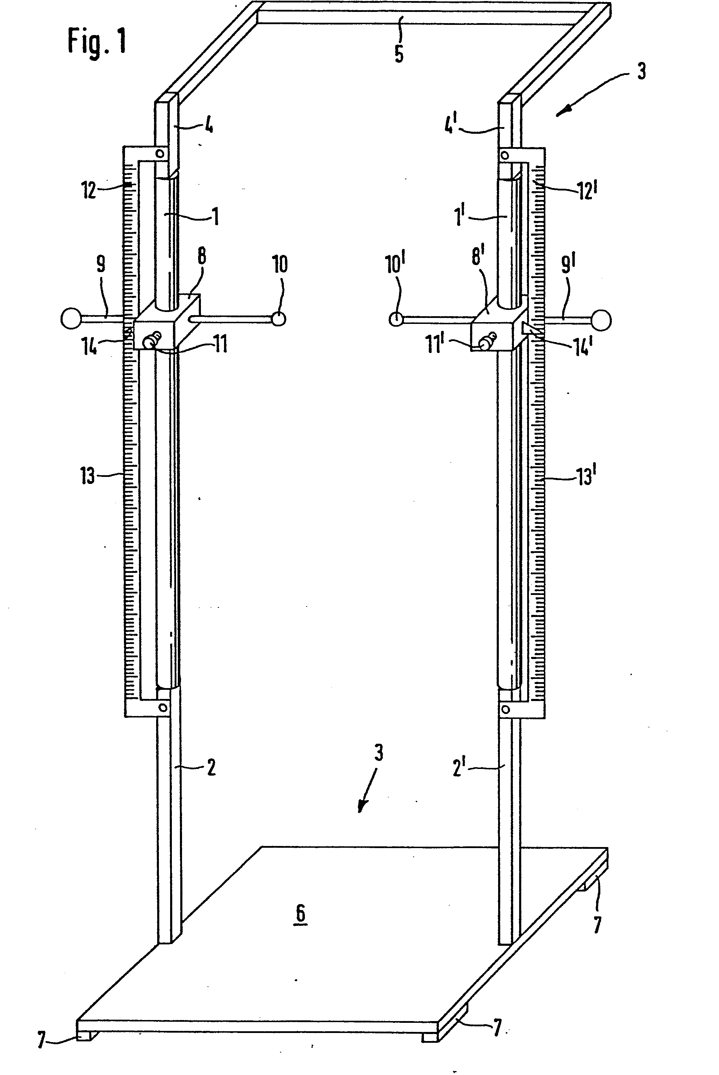

Bei der in Fig. 1 dargestellten Ausführungsform sind zwei
vertikale Führungen 1 und 1' vorgesehen, die über zwei zugeordnete Verlängerungs-Stangen 2 und 2' an einer Bodenplatte 6 befestigt sind. Der Abstand der Führungen 1 und 1' ist dabei etwas
größer als die Körperbreite des Patienten. Am oberen Ende der
Führungen schließen sich weitere Verlängerungs-Stangen 4 und 4'
an, an deren Ende ein U-förmiger Bügel 5 angebracht ist. Auf diese Weise bilden die beiden Führungen 1 und 1' einen Teil eines
Gestellrahmens 3, der ggfs. zur Erhöhung seiner Stabilität noch
mit Hilfe des Bügels 5 an einer Wand befestigt werden kann. Zur
Justierung des Gestellrahmens derart, daß die beiden Führungen 1
und 1' sich in der senkrechten Lage befinden, ist die Bodenplatte
6 zweckmäßig mit höhenverstellbaren Füßen 7 versehen.

Auf jeder Führung 1 und 1' ist eine stufenlos höhenverstellbare Halterung 8 bzw. 8' angeordnet, welche die Form eines umgreifenden Reiters besitzt und mittels Arretierschrauben 11 bzw.
11' auf der Führung feststellbar ist. Beide Halterungen enthalten
jeweils ein Lager für einen horizontal in Richtung auf die gegenüberliegende Führung hin und her längsverschieblichen Taststab 9
bzw. 9', der an seinem nach innen weisenden Ende einen Tastkopf
10 bzw. 10' trägt. Zweckmäßig ist jeder Taststab auch um seine
zugeordnete Führung horizontal verschwenkbar, was sich im einfachsten Fall dadurch bewirken läßt, daß die Führungen 1 und 1'
zylindrisch ausgebildet und die Halterungen 8 und 8' auf den
Führungen drehbar sind. Im übrigen ist jede Halterung mit einem

Zeiger 14 bzw. 14' versehen, der an einem Skalenträger 12 bzw.
12' mit einer Meßskala 13 bzw. 13' anliegt. Die beiden Meßskalen
13 und 13' brauchen nicht metrisch zu sein und können auch auf
einen beliebigen Nullpunkt bezogen werden. Sie müssen aber kongruent sein, so daß sie bei gleicher Höhenstellung der beiden
Tastköpfe 10 und 10' übereinstimmende Höhenwerte anzeigen.

Mit der Vorrichtung gemäß Fig. 1 läßt sich eine Meßreihe
beispielsweise folgendermaßen durchführen: Der Patient betritt
die (waagerecht einjustierte) Bodenplatte 6 zwischen den beiden
Führungen 1 und 1', und zwar dem Bügel 5 zugewendet mit locker
herabhängenden Armen. Die Bedienungsperson ergreift sodann mit je
einer Hand einen der beiden Taststäbe 9 und 9' nahe den Tastköpfen 10 und 10' und führt die Tastköpfe durch entsprechende Höhenverschiebung der Halterungen 8 und 8' und Längsverschiebung sowie
ggfs. Horizontalverschwenkung der Taststäbe 9 und 9' zunächst an
die beiden Trochanter heran. Danach wird die Höhenlage der beiden
Zeiger 14 und 14' abgelesen und notiert. Während dieses Vorgangs
können die Halterungen 8 und 8' mittels der Arretierschrauben 11
und 11' an den Führungen 1 und 1' arretiert werden. Anschließend
werden die Tastköpfe dann in gleicher Weise an die weiteren
Bezugspunkte herangeführt, um die Meßwerte für die Becken-Höhe
und die Schulterblatt-Höhe zu erhalten.

Vorteilhaft ist jeder Taststab 9 und 9' in Richtung auf die
gegenüberliegende Führung, also in Richtung nach innen federbelastet, was zeichnerisch nicht weiter dargestellt ist. Mit dieser
Federbelastung der Taststäbe läßt sich das Heranführen der Tastköpfe 10 und 10' an die jeweiligen Bezugspunkte unterstützen und
vereinfachen. Außerdem liegen dann in der Meßstellung die beiden
Tastköpfe beidseitig mit Druck am Körper des Patienten an, was
die Ruhighaltung des Patienten und auch die Fixierung der Meßstellung erleichtert. Im Falle einer solchen Federbelastung ist
es zweckmäßig, die Taststäbe 9 und 9' in einer äußeren, von der
gegenüberliegenden Führung wegweisenden Endlage arretierbar zu
machen, um dem Patienten ausreichend Raum zum Zutritt in den Zwischenraum zwischen den Führungen zu belassen. Eine einfache
Arretiermöglichkeit läßt sich bei zylindrischen Taststäben beispielsweise dadurch schaffen, daß an jedem Taststab ein kleiner
Haken angebracht wird, der in eine Öse auf der Innenseite der
zugeordneten Halterung eingreift und durch leichte Drehung des
Taststabes entriegelt werden kann.

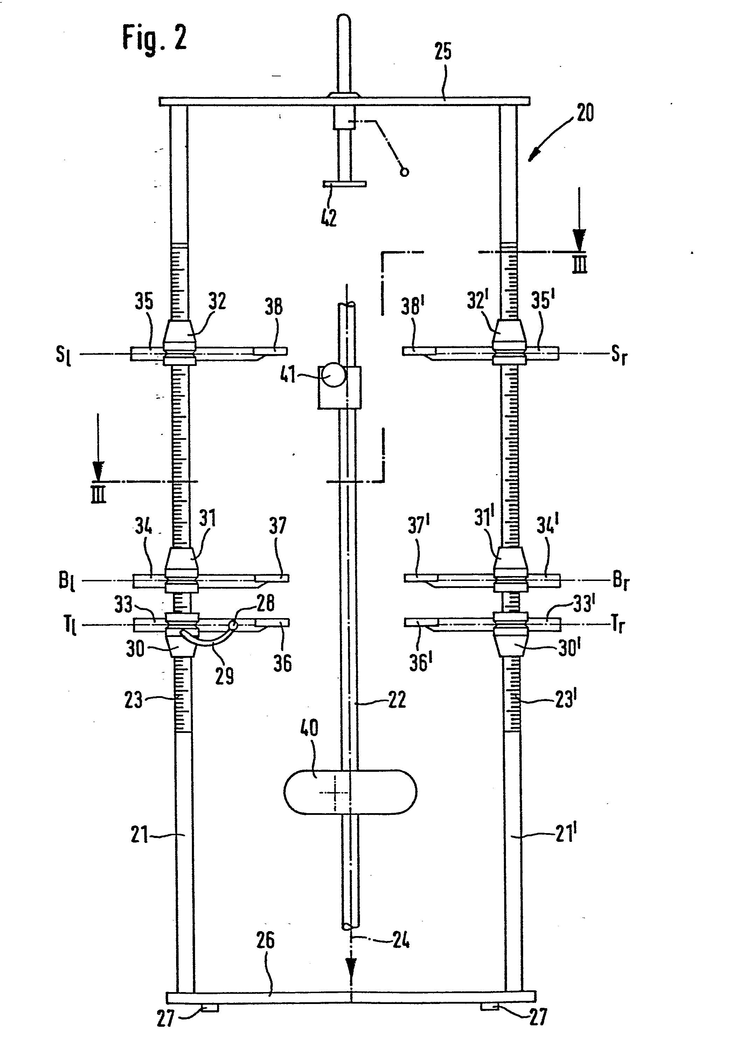

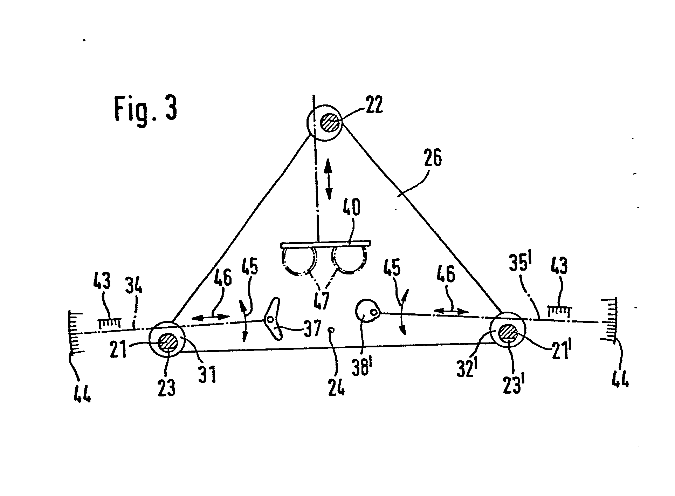

Die in den Fig. 2 und 3 dargestellte Ausführungsform entspricht in Aufbau und Funktion grundsätzlich der Ausführungsform
gemäß Fig. 1, weist jedoch für jedes Bezugspunkt-Paar $T_l$ und $T_r$,
$B_l$ und $B_r$ sowie $S_l$ und $S_r$ ein gesondertes Taststab-Paar auf,
sodaß jeder Taststab nach erfolgter Abtastung seines zugeordneten
Bezugspunktes in der Meßstellung belassen werden kann. Außerdem
sind bei dieser Ausführungsform noch zusätzliche Meßmöglichkeiten
gegeben.

Bei der Ausführungsform gemäß Fig. 2 und 3 erstrecken sich
zwei vertikale Führungen 21 und 21' zwischen einer im Grundriß
dreieckigen Bodenplatte 26 und einer ebensolchen Kopfplatte 25.
Ein zusätzlicher Stützpfosten 22 ergänzt die beiden Führungen und
die beiden Platten zu einem standfesten Gestellrahmen 20, der
mittels höhenverstellbarer !üße 27 justierbar ist.

An jeder Führung 21 und 21' sind drei höhenverschiebliche
und um ihre Führung drehbare Halterungen 30, 31 und 32 bzw. 30',
31' und 32' angeordnet, die ebenso wie in Fig. 1 ein Lager für je
einen horizontalverschieblichen Taststab 33, 34 und 35 bzw. 33',
34' und 35' bilden. Die Höhenlage der Halterungen ist an Skalen
23 bzw. 23' ablesbar, die sich wie in Fig. 1 an gesonderten
Skalenträgern befinden können, hier aber als in einer Ausnehmung
der Führungen angebracht gezeigt sind. Die Taststäbe der Paare 33
und 33', 34 und 34' sowie 35 und 35' können in diesem Fall wegen
ihrer Zuordnung zu vorbestimmten Bezugspunkten mit einem jeweils
an die Anatomie der betreffenden Bezugspunkte angepaßten Tastkopf
36 und 36', 37 und 37' sowie 38 und 38' versehen sein, um eine
gute Erfassung des jeweiligen Knochenteiles zu gewährleisten.

Alle Halterungen sind auf ihrer Führung arretierbar, um sie in der erreichten Meßstellung feststellen zu können. Dazu können wie in Fig. 1 Arretierschrauben oder Klemmhebel an den Halterungen vorgesehen sein, aber bevorzugt wird hier eine (schematisch am Beispiel der Halterung 30 gezeigte und für die übrigen Halterungen analog geltende) fernbediente Arretierung eingesetzt, deren Auslösung 28 sich an dem Taststab 33 nahe dem Tastkopf 36 befindet und in geeigneter Weise, beispielsweise mittels eines Bowdenzuges oder eines elektrischen Kabels 29 mit einer in der Halterung befindlichen Arretiereinrichtung verbunden ist. Dadurch braucht die Bedienungsperson nach dem Erreichen der Meßstellung den Taststab nicht mehr loszulassen, um die Halterung zu arretieren. Besonders günstig ist es dabei, auch die Taststäbe in der Meßstellung arretierbar zu machen, was sich z.B. dadurch erzielen läßt, daß die fernbediente Arretiereinrichtung sowohl die Halterung an der Führung als auch den Taststab in der Halterung feststellt. Im übrigen können die Taststäbe zweckmäßig auch hier in der schon anhand von Fig. 1 erläuterten Weise in Richtung nach innen federbelastet sein.

Mit einer fernbedienten Arretierung gelingt die Durchführung einer Meßreihe besonders schnell und sicher. Die Bedienungsperson ergreift jeweils ein Taststab-Paar, entarretiert durch Druck auf die Auslösungen 28 die Halterungen (ggfs. zusammen mit den Taststäben) und führt anschließend die Tastköpfe in die Meßstellung. Danach braucht sie nur noch die Taststäbe loszulassen, wodurch sofort wieder die Arretierung der Halterungen (und ggfs. der Taststäbe) wirksam wird, ohne daß die Bedienungsperson noch weitere Handgriffe ausführen muß. Sie kann sich also ganz auf die Durchführung der Messungen konzentrieren.

Das Belassen der Tastköpfe in der Meßstellung während der Durchführung einer Meßreihe unterstützt die Ruhighaltung des Patienten. Zusätzlich können aber noch weitere Hilfsmittel zur Ruhighaltung vorgesehen sein, nämlich z.B. eine Kniestütze 40, eine Bruststütze 41 und eine Kopfstütze 42. Die Kniestütze und die Bruststütze können dabei in eine für jeden Patienten individuelle Position einstellbar an dem Stützpfosten 22 befestigt sein, während die Kopfstütze verstellbar in der Kopfplatte 25 gelagert ist und ggfs. zugleich zur Größenmessung des Patienten herangezogen werden kann.

Mit der Ausführungsform der Fig. 2 und 3 lassen sich auch eine Beckenrotation und ein seitlicher Beckenversatz messen, wenn mindestens dem Taststab-Paar 34 und 34', zweckmäßig aber allen Taststäben zusätzlich noch Skalen 43 und 44 zur quantitativen Erfassung des jeweiligen Schwenkwinkels 45 sowie des Längsverschiebungsweges 46 zugeordnet werden, wie dies in Fig. 3 schematisch angedeutet ist. Hierbei ist ein Hilfsmittel zur Fixierung des Patienten notwendig, wofür zweckmäßig die Kniestütze 40 herangezogen wird, die ggfs. auch noch mit Riemen 47 zum Umgreifen und Festlegen der Kniepartie versehen sein kann.

In allen Ausführungsformen der Erfindung kann das Ablesen der Skalen visuell erfolgen. Ebenso kann aber auch eine optische oder elektronische Meßwertabtastung der Skalen vorgesehen sein, die ggfs. mit einer (auch dem Patienten sichtbaren) Fernanzeige und/oder einer Datenspeicherung gekoppelt ist. Dies ist zeichnerisch nicht weiter dargestellt.

Im übrigen kann es in vielen Fällen wünschenswert sein, innerhalb der Vorrichtung noch ein Lot 24 (das ggfs. auch die Form eines straff zwischen zwei Ösen einhängbaren Gummibandes haben kann) vorzusehen, um den Patienten vor Beginn der Messungen durch Augenschein abschätzen zu können und um die Positionierung des Patienten in der Vorrichtung zu erleichtern.

Patentansprüche

—

1.      Vorrichtung zur Messung einer Fehlhaltung des Beckens,
der Schulter sowie der Wirbelsäule des menschlichen Körpers,
dadurch gekennzeichnet, daß zwei vertikale Führungen im Abstand
von mehr als einer Körperbreite vorgesehen sind, daß auf jeder
Führung mindestens eine höhenverschiebbare Halterung angeordnet
ist, deren jeweilige Höhenstellung quantitativ erfaßbar ist, und
daß in jeder Halterung ein in den Zwischenraum zwischen den
Führungen hineinragender horizontaler Taststab zur Abtastung
ausgewählter Bezugspunkte des Knochengerüstes längsverschieblich
gelagert ist.

2.      Vorrichtung nach Anspruch 1, dadurch gekennzeichnet,
daß die Taststäbe um die zugeordnete Führung in der Horizontalebene schwenkbar sind.

3.      Vorrichtung nach Anspruch 1 oder 2, dadurch gekennzeichnet, daß die Höhenstellung der Halterungen auf ihrer Führung
arretierbar ist.

4.      Vorrichtung nach einem der Ansprüche 1 bis 3, dadurch
gekennzeichnet, daß die Längsverschiebung der Tastköpfe in ihrer
Halterung arretierbar ist.

5.      Vorrichtung nach einem der Ansprüche 1 bis 4, dadurch
gekennzeichnet, daß die Taststäbe in Richtung auf den Zwischenraum zwischen den Führungen federbelastet sind.

6.      Vorrichtung nach einem der Ansprüche 1 bis 5, dadurch
gekennzeichnet, daß zusätzliche Stütz- und Haltemittel zur Ruhighaltung des Körpers vorgesehen sind.

7.      Vorrichtung nach Anspruch 6, dadurch gekennzeichnet,
daß die Stütz- und Haltemittel eine Kniestütze umfassen.

8.      Vorrichtung nach einem der Ansprüche 1 bis 7, dadurch gekennzeichnet, daß die horizontale Längsverschiebung und die horizontale Verschwenkung der Taststäbe quantitativ erfaßbar ist.

9.      Vorrichtung nach einem der Ansprüche 1 bis 8, dadurch gekennzeichnet, daß pro Führung zwei oder drei Halterungen mit je einem darin längsverschieblich gelagerten Taststab vorgesehen sind.

10.      Vorrichtung nach Anspruch 9, dadurch gekennzeichnet, daß jeder Taststab an seinem inneren Ende mit einem anatomisch an den abzutastenden Bezugspunkt angepaßten Tastkopf versehen ist.

Fig. 1

1/3

0144528

Fig. 2

Fig. 3

0144528

3/3